# EUROPEAN PATENT SPECIFICATION

(11) **EP 2 714 669 B1**
(45) Date of publication and mention of the grant of the patent: **26.04.2017**
(21) Application number: 12789268.5
(22) Date of filing: 22.05.2012
(51) Int. Cl.: C07D 285/24, C07D 249/12, A01N 37/44, A61P 3/00

(54) **HYPERTENSION AND HYPERURICEMIA**
HYPERTONIE UND HYPERURIKÄMIE
HYPERTENSION ET HYPERURICÉMIE

(30) Priority: 24.05.2011 US 201161489597 P
(43) Date of publication of application: 09.04.2014
(73) Proprietor: Ardea Biosciences, Inc., San Diego CA 92121 (US)
(72) Inventor: MINER, Jeffrey, San Diego, CA 92122 (US)
(74) Representative: HGF Limited
(86) International application number: PCT/US2012/039011
(87) International publication number: WO 2012/162323

(56) References cited:
- US-A1- 2009 054 319
- US-A1- 2009 197 825
- US-A1- 2010 056 464
- BURNS ET AL: "Gout therapeutics; New drugs for and old disease", THE LANCET, vol. 377, 17 August 2010 (2010-08-17), - 17 August 2010 (2010-08-17), pages 165-177, XP002732653,
- VALLON ET AL.: 'Overlapping in vitro and in vivo specificities of the organic anion transporters OAT1 and OAT3 for loop and thiazide diuretics.' AMERICAN JOURNAL OF PHYSIOLOGY. RENAL PHYSIOLOGY vol. 294, no. 4, 23 January 2008, pages F867 - 873, XP055135807 Retrieved from the Internet: <URL:http://www.ncbi.nlm.nih.gov/pubmed> [retrieved on 2012-08-01]

## Description

### CROSS-REFERENCE

This application claims the benefit of U.S. Provisional Application No. 61/489,597, filed May 24, 2011.

### BACKGROUND OF THE INVENTION

Hypertension is treated with anti-hypertensive agents such as thiazide diuretics, which may elevate serum uric acid levels.

### SUMMARY OF THE INVENTION

In a first embodiment, provided herein is Lesinurad or a pharmaceutically acceptable salt thereof and allopurinol for use in the treatment of gout in a patient also being treated with a thiazide diuretic.

Described herein is a method for treating hypertension in a subject in need thereof (e.g., wherein said treatment does not result in an increase in serum uric acid levels, does not result in abnormally elevated serum uric acid levels, does not result in hyperuricemia, does not result in serum uric acid levels of above 6 mg/dL, or does not result in the development of gout in the subject), the method comprising administering to the subject:
a. a thiazide diuretic; and
b. an organic anion transporter 4 (OAT4) inhibitor.

In some embodiments, the thiazide diuretic is selected from hydrochlorothiazide, bendroflumethiazide, benzothiadiazine, hydroflumethiazide, clorothiazide, methyclothiazide, polythiazide, chlorthalidone, metolazone, indapamide, bumetanide, ethacrynic acid, furosemide or torsemide.

The OAT4 inhibitor is 2-(5-bromo-4-(4-cyclopropylnaphthalen-1-yl)-4*H*-1,2,4-triazol-3-ylthio)acetic acid or a pharmaceutically acceptable salt thereof.

In some embodiments, any method described herein further comprises administering a URAT1 inhibitor to the subject. In specific embodiments, the OAT4 inhibitor and the URAT1 inhibitor are the same drug.

Desribed herein is a method for treating hypertension in a subject in need thereof, wherein said treatment does not result in an increase in serum uric acid levels, comprising administering to the subject:
a. hydrochlorothiazide; and
b. 2-(5-bromo-4-(4-cyclopropylnaphthalen-l-yl)-4*H*-1,2,4-triazol-3-ylthio)acetic acid or a pharmaceutically acceptable salt thereof

Provided in some embodiments herein is Lesinurad or a pharmaceutically acceptable salt thereof and allopurinol for use in reducing serum uric acid, treating gout, or reducing the incidences of elevated serum uric acid or gout in a subject suffering from hypertension, wherein said subject is also being treated with a thiazide diuretic. In specific embodiments, the elevated serum uric acid or gout is induced by the administration of a thiazide. In certain embodiments, provided herein is Lesinurad or a pharmaceutically acceptable salt thereof and allopurinol for use in treating an OAT4 mediated disorder in a subject. In specific embodiments, the OAT4 mediated disorder is OAT4 mediated hyperuricemia or OAT4 mediated gout.

In some embodiments, provided herein is Lesinurad or a pharmaceutically acceptable salt thereof and allopurinol for use in reducing the incidences of or likelihood of or reversing the diagnosis of hyperuricemia or gout in a patient receiving thiazide treatment.

In certain embodiments, provided herein is Lesinurad or a pharmaceutically acceptable salt thereof and allopurinol for use in reducing serum uric acid levels in a patient suffering from hypertension, wherein the patient is receiving a thiazide diuretic, and wherein (absent the administration of the OAT-4 inhibitor) administration of the thiazide diuretic results in elevated serum uric acid levels.

In some embodiments, provided herein is a composition comprising:
a. a thiazide diuretic;
b. Lesinurad or a pharmaceutically acceptable salt thereof; and
c. a pharmaceutically acceptable excipient or carrier.

In specific embodiments, provided herein is a composition comprising:
a. hydrochlorothiazide;
b. 2-(5-bromo-4-(4-cyclopropylnaphthalen-1-yl)-4*H*-1,2,4-triazol-3-ylthio)acetic acid or a pharmaceutically acceptable salt thereof; and
c. a pharmaceutically acceptable excipient or carrier.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIGURE 1** represents a pictorial representation of two mechanisms for Hydrochlorothiazide (HCTZ)-induced hyperuricemia; a) Direct - HCTZ enhancement of Uric acid uptake by OAT4; and b) Indirect - HCTZ enhances an OAT4 stimulatory protein (NHE3).
**FIGURE 2** represents OAT4 transport activity of 6-carboxyfluorescein (CF) substrate incubated with 50µM Lesinurad (black) or vehicle (light grey) in HEK293T cells transiently transfected with either (a) control plasmid lacking OAT4 (pCMV) or (b) OAT4.
**FIGURE 3** represents HEK293T cells transiently transfected with OAT4 (grey) or control plasmid lacking OAT4 (pCMV, black) incubated with 6-carboxyfluorescein (CF) substrate with various amounts (0, 0.5, 1, 2nM) cold uric acid illustrating urate acts as a competitive substrate for OAT4 of CF (EC₅₀ ∼900µM).
**FIGURE 4** represents the amount of OAT4 urate transport (cpm) in the presence of varying amounts of Lesinurad (■) and benzbromarone (▲). The OAT4 IC₅₀ of Lesinurad =5µM; and Benzbromarone = 10µM.
**FIGURE 5** represents the percent inhibition of UA transport in 293T cells expressing URAT1 and/or OAT4 by Lesinurad at varying concentrations, indicating Lesinurad inhibits URAT1 and OAT4 with similar potency.
**FIGURE 6** represents percent ³H-Estrone sulphate (ES) transport in 293T cells stably expressing OAT4, in the presence (-) or absence (- - - -) of 1mM Hydrochlorothiazide (HCTZ), and varying concentrations of Lesinurad, indicating HCTZ has no effect on Lesinurad-mediated inhibition of OAT4 transporter activity.
**FIGURE 7** represents the OAT4 transport activity of (a) 6-carboxyfluorescein (CF-5µM) and (b) ¹⁴C-uric acid (UA - 100µM) substrates in the presence of vehicle, Lesinurad, oxypurinol, or allopurinol (100µM - 5 min incubation) in OAT4-expressing HEK293 cells indicating oxypurinol and allopurinol do not inhibit OAT4 transport activity.
**FIGURE 8** represents percent uptake of ³H-Estrone sulfate (ES) in OAT4-expressing oocytes injected with various concentrations of Lesinurad (25, 50, 100µM - outside; 22, 44, 444µM - inside) or vehicle, indicating Lesinurad inhibits OAT4 primarily from the extracellular (apical) side.
**FIGURE 9** represents the amount of ¹⁴C-labeled Lesinurad (measured by scintillation counting) inside and outside OAT4-expressing oocytes after being injected with Lesinurad (50nL) and incubated for 30 mins, indicating Lesinurad remains inside injected oocytes for the duration of the experiment.
**FIGURE 10A** represents a schematic of a clinical phase 2 study design.
**FIGURE 10B** represents the proportion of patients with serum uric acid (sUA) levels below 6mg/dL, separated into those taking diuretics (black) and those not taking diuretic (light grey) for the various doses of Lesinurad, and indicating patients receiving diuretics had responded well to Lesinurad.

### DETAILED DESCRIPTION OF THE INVENTION

While certain embodiments of the present invention have been shown and described herein, it will be obvious to those skilled in the art that such embodiments are provided by way of example only. Numerous variations, changes, and substitutions will occur to those skilled in the art without departing from the invention. It should be understood that various alternatives to the embodiments described herein are, in some circumstances, employed in practicing the invention. It is intended that the following claims define the scope of the invention and that methods and structures within the scope of these claims and their equivalents be covered thereby.

### Diuretics and Hyperuricemia

Hypertension is prevalent in gout patients and they are often treated with anti-hypertensive agents, such as thiazide diuretics, which have been known since the 1950's to elevate serum uric acid levels (Healey et. al., NEJM, 1959, 261, 1358). For example, one study revealed use of thiazide diuretics in doses of 25 mg/day or higher is associated with a significantly increased risk for initiation of anti-gout therapy (Gurwitz et. al., J Clin. Epidemiol. 1997, 50(8), 953).

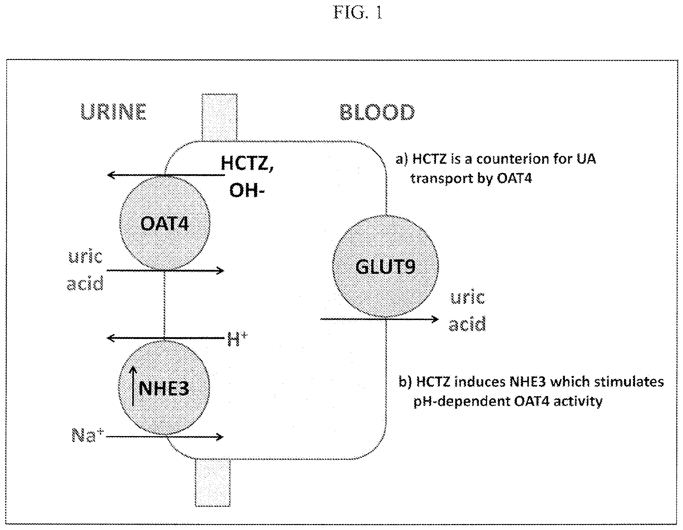

Meanwhile, urate-lowering therapies are generally believed to work less efficiently in patients taking diuretics concomitantly (Reyes, Cardiovasc. Drugs Ther., 2003, 17(5-6), 397). This effect is thought to be mediated by enhanced uric acid reabsorption due to activation of OAT4 by two different mechanisms (see Figure 1); either direct thiazide enhancement of uric acid uptake by OAT4 (Hagos et al, J. Am. Soc. Nephrol., 2007, 18, 430), or indirect via thiazide enhancement of an OAT4 stimulatory protein (Sodium/hydrogen exchanger 3; NHE3) (Nijenhuis et. al., J. Clin. Invest. 2005, 115, 1651).

In addition to URAT1, organic anion transporter 4 (OAT4) is considered an important regulator of urate excretion. Organic anion transporter 4 (OAT4) is a urate transporter, also involved in renal secretion of anti-hypertensive drugs such as thiazide diuretics. In some instances, OAT4 exchanges these drugs against urate, thereby enhancing uric acid reabsorption resulting in their hyperuricemic effect. It has been postulated that OAT4 may be responsible for the hyperuricemia associated with some diuretics.

In some instances, diuretic use is linked to increase risk of gout and increased serum uric acid levels. (Arch. Intern. Med. 2005; 165: 742-8.) Indeed, all loop diuretics, and many thiazide-type diuretics elevate serum urate, with the exception of tienilic acid, (which is uricosuric and reduces serum urate). Sodium channel blockers (such as amiloride and triamterene) and aldosterone receptor blockers (such as spironolactone and eplerenone) also elevate serum urate levels, though the mechanisms are probably different for each drug class. Fractional excretion of urate is reduced in diuretic treated subjects (J. Am. Soc. Nephrol. 18:3101, 2007). In some instances, mechanisms that may explain these observations include possible volume effects of diuretics, inhibition of urate secretion transporters in proximal tubule (NPT), and direct or indirect activation of OAT4 (SLC22A11) and URAT1. (Reyes, Cardiovascular Drugs and Serum Uric Acid, Cardiovascular Drugs and Therapy 17:397, 2004.)

In some instances, diuretics function as counterion substrates, secreted into the urine by OAT4, which promotes reabsorption of uric acid. For examples, Hydrochlorothiazide (J. Am. Soc. Nephrol. 18:430, 2007) and torasemide (J. Am. Soc. Nephrol. 18:3101, 2007) increase OAT4 uric acid transport activity.

Genetic evidence supports the role of OAT4 and URAT1 in gout, hyperuricemia and OAT4 diuretic induced hyperuricemia, whereby two association studies show OAT4 independent associations (Circ. Cardiovasc. Genet. 2010; 3; 523-530 and Kolz et al, 2009. Vol 5:6), and another study shows OAT4 association with diuretic-induced hyperuricemia (McAdams presentation ACR Arthritis & Rheumatism, Volume 63, November 2011 Abstract Supplement).

In some instances, fluid volume alterations are a dominant effect of diuretics through blockade of sodium transporters, and urate absorption parallels NaCl absorption by the proximal tubule. Iron urate and serum uric acid levels correlate well with volume status. Hyperuricemia is abrogated by salt loading the diuretic treated patients, consistent with volume status playing an important role. (2 mg/dl decrease in SUA between hypertensive patients on ∼20 mequ/day salt restriction versus 250 mEqu/day salt loading, with hyperuricemia in the salt-deprived patients.) In some instances, diuretics cause decreased fractional excretion of urate where the volume effects on sUA could be due to alterations in sodium and proton balance in proximal tubule cells leading to activation of URAT1 and OAT4. (Am. J. Physiol. 1996 Nov; 271(5 Pt 2):F1093-9 , Nijenhuis et al, J. Clin. Invest. 115:1651, 2005; J. Am. Soc. Nephrol. 18: 3101-3109, 2007).

One study (Arthritis & Rheumatism, Volume 63, November 2011 Abstract Supplement, Abstracts of the Am Coll of Rheumatology/Assoc of Rheumatology Health Professionals Annual Scientific Meeting) concluded that the increased risk of gout related to diuretic use in hypertensive subjects was only observed among those with a higher genetic risk score for elevated serum urate levels, suggesting a urate gene-by-diuretic interaction, delineating an important interaction of genetic traits influencing urate metabolism and handling with diuretic use in hypertensive subjects.

### Lesinurad,

Lesinurad is the generic name for 2-(5-bromo-4-(4-cyclopropylnaphthalen-1-yl)-4H-1,2,4-triazol-3-ylthio)acetic acid, whose chemical structure is:

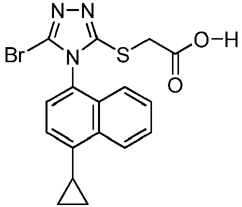

In some instances, the term Lesinurad also includes the sodium salt of Lesinurad, i.e. sodium 2-(5-bromo-4-(4-cyclopropylnaphthalen-1-yl)-4H-1,2,4-triazol-3-ylthio)acetate.

Lesinurad is a urate lowering therapy in clinical development for the treatment of gout. In some instances, Lesinurad blocks reabsorption of urate (UA) within the kidney proximal tubule by inhibiting the URAT1 transporter.

### Thiazides / thiazide diuretics

Thiazides or thiazide diuretics are used to treat hypertension (high blood pressure) and edema (such as that caused by heart, liver, or kidney disease), reducing the risk of death, stroke, heart attack and heart failure due to hypertension. Thiazides are the most commonly used diuretic as the recommended first-line treatment in the US and a recommended treatment in the Europe. Thiazides are generally understood to work by inhibiting reabsorption of sodium and chloride ions from the distal convoluted tubules in the kidneys, by blocking the thiazide-sensitive Na⁺-Cl⁻ symporter, resulting in increased sodium excretion and thereby increased water excretion, i.e. increasing urination. In some instances, decreasing the amount of water in the body may result in a lower blood volume, thereby reducing and cardiac output, and ultimately leading to a fall in arterial pressure.

The term "thiazide" refers to a drug acting at a "thiazide receptor", and includes "thiazide-like diuretics" which act similarly to thiazides but do not contain the benzothiadiazine molecular structure. Examples of "thiazide-like diuretics" include, but are not limited to, bendroflumethiazide, benzthiazide, chlorothiazide, hydrochlorothiazide, hydroflumethiazide, indapamide, methyclothiazide, polythiazide, quinethazone, trichlormethiazide, chlortalidone and metolazone.

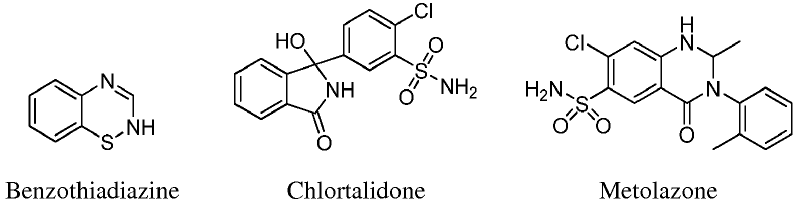

### Hydrochlorothiazide

Hydrochlorothiazide, 6-Chloro-1,1-dioxo-3,4-dihydro-2*H*-1,2,4-benzothiadiazine-7-sulfonamide (HCTZ, HCT or HZT) is frequently prescribed for treatment of hypertension and congestive heart failure.

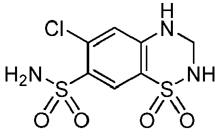

Hydrochlorothiazide is generally understood to act on the kidneys to reduce sodium reabsorption and inhibiting the kidneys' ability to retain water, thereby reducing blood volume, decreasing blood return to the heart and thus cardiac output. Hydrochlorothiazide competes for the chloride site on a Na⁺/Cl⁻ co-transporter, thereby impairing sodium transport. One study observed that use of hydrochlorothiazide resulted in a 2.6-fold increase of OAT4-mediated uric acid uptake.

### Organic Anion Transporter-4 (OAT4)

Human organic anion transporter 4 (hOAT4) is expressed in the kidney and, in some instances, encodes a 550 amino acid residue protein. In some instances, hOAT4 is involved in renal secretion and reabsorption of endogenous substances as well as many drugs and xenobiotics. Generally, OAT4 may be present at the luminal side membrane of the proximal renal tubule and mediates the transport of organic anions such as esteron sulfate (ES), dehydroepiandrosterone (DHEA) sulfate, and ochratoxin A, p-aminohippurate (PAH).

Benzbromarone and 6-hydroxybenzbromarone are inhibitors of OAT4, (which thereby promote uric acid excretion). Benzbromarone inhibits OAT4 uptake of ³H-estrone sulfate with an IC50 of 5.4 µmol/L, and 6-hydroxybenzbromarone inhibits OAT4 uptake of ³H-estrone sulfate with an IC50 of 3.2 µmol/L.

### Examples

The examples and preparations provided below further illustrate and exemplify the compounds of the present invention and methods of preparing such compounds. It is to be understood that the scope of the present invention is not limited in any way by the scope of the following examples and preparations.

The urate transporter OAT4 was stably expressed in cultured cells and oocytes.

For cultured cells, HEK293 cells stably expressing the transporters were produced through transfection of DNA constructs carrying the transporters, antibiotic selection, and clonal selection of clones with high transporter activity. Alternatively, cells transiently expressing the transporters were produced by reverse transfection of HEK293T cells. Transfectants were plated at high density onto poly-L-lysine coated multiwell plates and assayed 1-2 days later. Results were similar for stable and transient expressing cells.

Oocytes were injected with cRNA for OAT4 expression and assayed 3-4 days later.

Activity assays were performed by incubating the cells with transporter substrates in assay buffer containing 125 mM sodium gluconate, 4.8 mM potassium gluconate, 1.2 mM monobasic sodium phosphate, 1.2 mM magnesium sulfate, 1.3 mM calcium gluconate, 5.6 mM glucose, and 25 mM HEPES pH 7.1. test drugs were added to the cells prior to addition of substrate for the indicated times. Substrates used were 6-carboxyfluorescein (CF) at 5 µM, 3H-estrone sulfate (ES) at 50 nM, and 14C-uric acid (UA) at 100 µM. For transfected cells, substrates were incubated for 2 minutes and then removed by aspiration and the cells washed three times in a wash buffer containing 125 mM sodium gluconate and 25 mM HEPES pH 7.1. Cells were then lysed in 1 M sodium hydroxide prior to fluorescence measurement for CF transport and liquid scintillation counting for ES and UA. Oocyte assays were performed similarly, except that test drugs were injected and then transport was measured after 30 minutes (ES) or 60 minutes (UA). The results of these assays are summarized in the figures as listed below.

**FIGURE 1** represents a pictorial representation of two mechanisms for Hydrochlorothiazide (HCTZ)-induced hyperuricemia; a) Direct - HCTZ enhancement of Uric acid uptake by OAT4; and b) Indirect - HCTZ enhances an OAT4 stimulatory protein (NHE3).

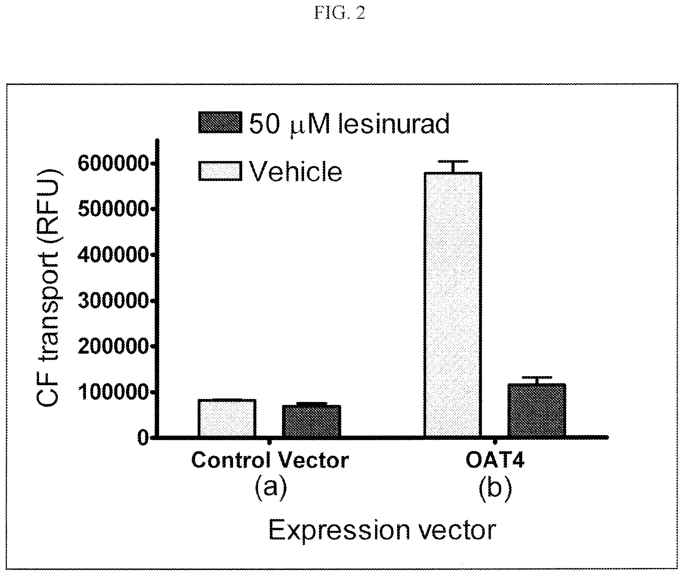

**FIGURE 2** represents OAT4 transport activity of 6-carboxyfluorescein (CF) substrate incubated with 50µM Lesinurad (black) or vehicle (light grey) in HEK293T cells transiently transfected with either (a) control plasmid lacking OAT4 (pCMV) or (b) OAT4.

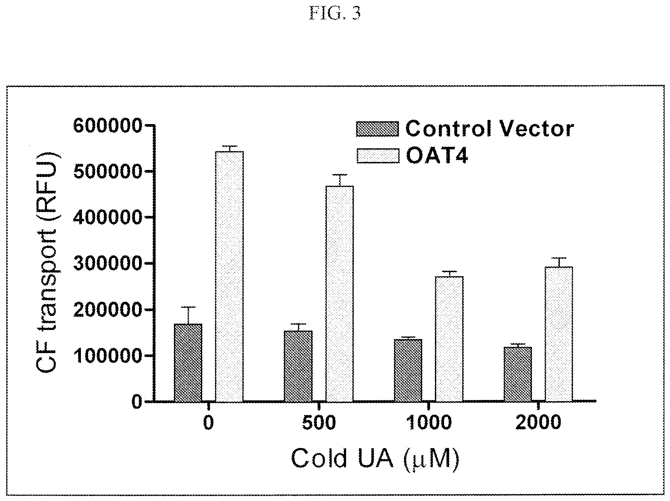

**FIGURE 3** represents HEK293T cells transiently transfected with OAT4 (grey) or control plasmid lacking OAT4 (pCMV, black) incubated with 6-carboxyfluorescein (CF) substrate with various amounts (0, 0.5, 1, 2nM) cold uric acid illustrating urate acts as a competitive substrate for OAT4 of CF (EC₅₀ ∼900µM).

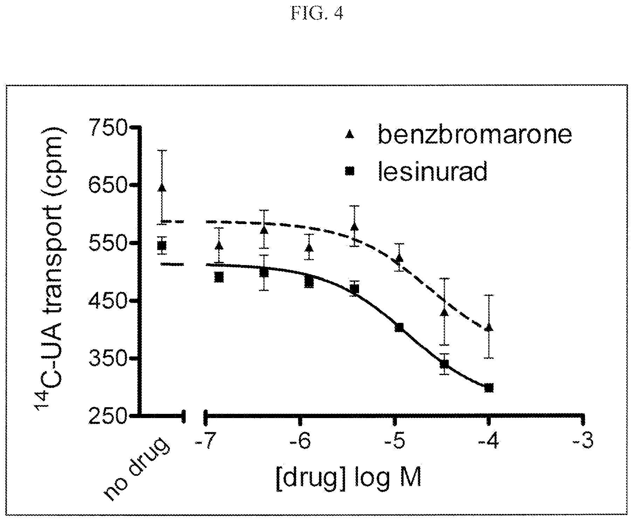

**FIGURE 4** represents the amount of OAT4 urate transport (cpm) in the presence of varying amounts of Lesinurad (■) and benzbromarone (▲). The OAT4 IC₅₀ of Lesinurad =5µM; and Benzbromarone = 10µM.

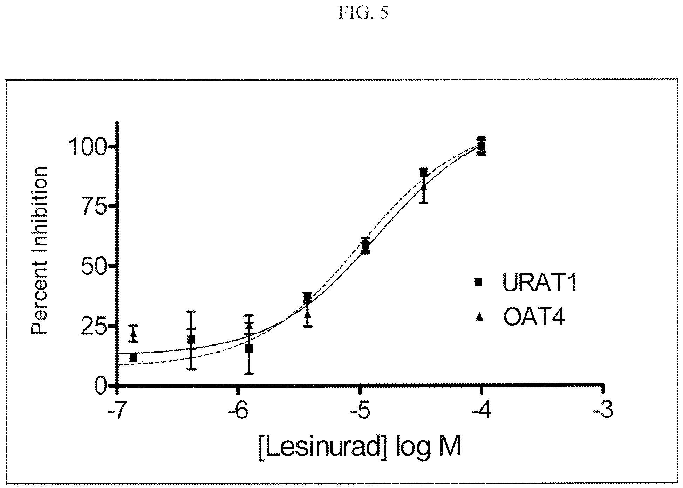

**FIGURE 5** represents the percent inhibition of UA transport in 293T cells expressing URAT1 and/or OAT4 by Lesinurad at varying concentrations, indicating Lesinurad inhibits URAT1 and OAT4 with similar potency.

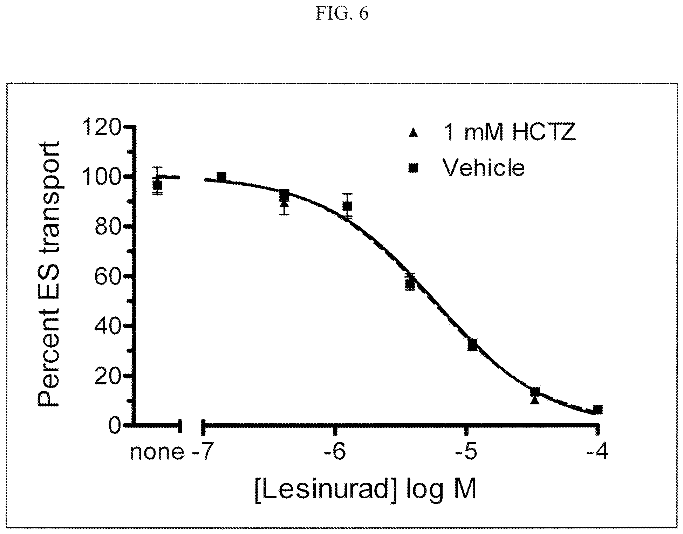

**FIGURE 6** represents percent ³H-Estrone sulphate (ES) transport in 293T cells stably expressing OAT4, in the presence (-) or absence (- - - -) of 1mM Hydrochlorothiazide (HCTZ), and varying concentrations of Lesinurad, indicating HCTZ has no effect on Lesinurad-mediated inhibition of OAT4 transporter activity.

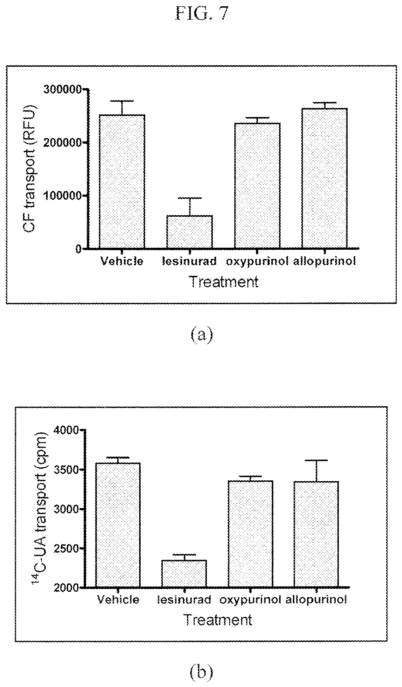

**FIGURE 7** represents the OAT4 transport activity of (a) 6-carboxyfluorescein (CF - 5µM) and (b) ¹⁴C-uric acid (UA - 100µM) substrates in the presence of vehicle, Lesinurad, oxypurinol, or allopurinol (100µM - 5 min incubation) in OAT4-expressing HEK293 cells indicating oxypurinol and allopurinol do not inhibit OAT4 transport activity.

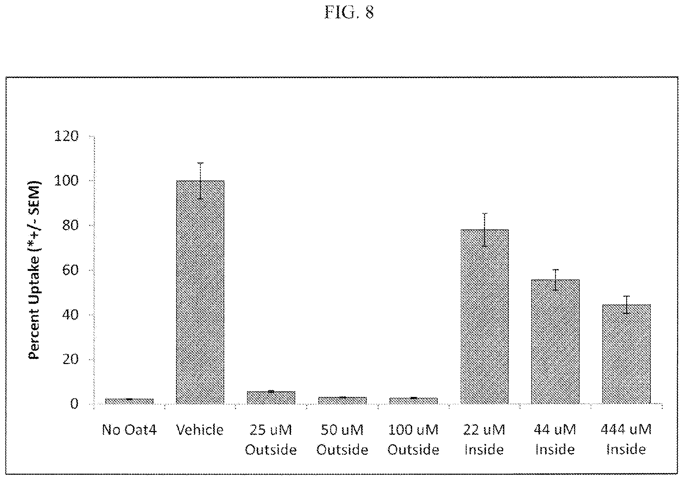

**FIGURE 8** represents percent uptake of ³H-Estrone sulfate (ES) in OAT4-expressing oocytes injected with various concentrations of Lesinurad (25, 50, 100µM - outside; 22, 44, 444µM - inside) or vehicle, indicating Lesinurad inhibits OAT4 primarily from the extracellular (apical) side.

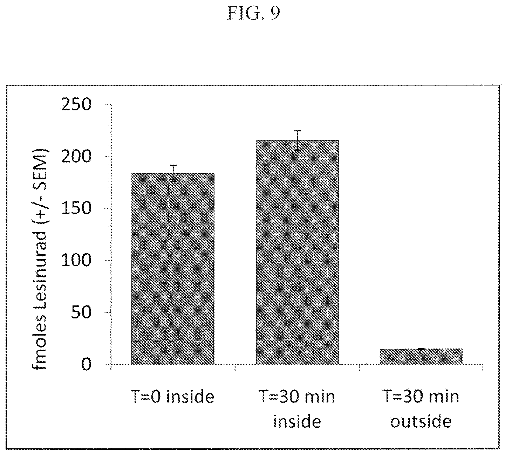

**FIGURE 9** represents the amount of ¹⁴C-labeled Lesinurad (measured by scintillation counting) inside and outside OAT4-expressing oocytes after being injected with Lesinurad (50nL) and incubated for 30 mins, indicating Lesinurad remains inside injected oocytes for the duration of the experiment.

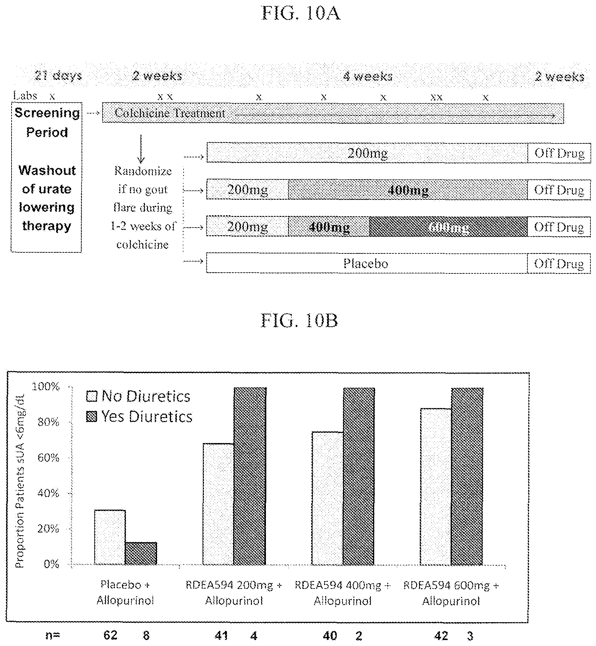

**FIGURE 10A** represents a schematic of a clinical phase 2 study design. The study was a 4-week, double-blind, placebo-controlled clinical trial in 208 gout patients who were not adequately responding to allopurinol with serum urate (sUA) ≥ 6 mg/dL while receiving a stable dose of allopurinol for at least 6 weeks. One of three doses of Lesinurad or matching placebo was added to the patients allopurinol regimen. The primary endpoint of the study was mean reduction in sUA at Week 4, with the key secondary endpoint the proportion of subjects with sUA < 6.0 mg/dL at Week 4. A small number of patients in this trial received a thiazide diuretic during the treatment period; their response rates were compared to those patients not receiving concomitant thiazide diuretics and the results presented in **FIGURE 10B****.** This figure represents the proportion of patients with serum uric acid (sUA) levels below 6mg/dL, separated into those taking diuretics (black) and those not taking diuretic (light grey) for the various doses of Lesinurad, and indicating patients receiving diuretics had responded well to Lesinurad.

## Claims

1. Lesinurad or a pharmaceutically acceptable salt thereof and allopurinol for use in the treatment of gout in a patient also being treated with a thiazide diuretic.

2. Lesinurad sodium and allopurinol for use as claimed in claim 1.

3. Lesinurad or a pharmaceutically acceptable salt thereof and allopurinol for use as claimed in claim 1 wherein the thiazide diuretic is hydrochlorothiazide.

4. Lesinurad sodium and allopurinol for use as claimed in claim 1 wherein the thiazide diuretic is hydrochlorothiazide.

5. Lesinurad or a pharmaceutically acceptable salt thereof for use in the treatment of gout in a patient also being treated with a thiazide diuretic and with allopurinol where treatment without Lesinurad or a pharmaceutically acceptable salt thereof would not be adequate.

6. Lesinurad sodium for use as claimed in claim 5.

7. Lesinurad or a pharmaceutically acceptable salt for use as claimed in claim 5 wherein the thiazide diuretic is hydrochlorothiazide.

8. Lesinurad sodium for use as claimed in claim 6 wherein the thiazide diuretic is hydrochlorothiazide.

## Patentansprüche

1. Lesinurad oder ein pharmazeutisch unbedenkliches Salz davon und Allopurinol zur Verwendung bei der Behandlung von Gicht in einem Patienten, der ebenfalls mit einem Thiaziddiuretikum behandelt wird.

2. Lesinurad-Natrium und Allopurinol zur Verwendung nach Anspruch 1.

3. Lesinurad oder ein pharmazeutisch unbedenkliches Salz davon und Allopurinol zur Verwendung nach Anspruch 1, wobei das Thiaziddiuretikum Hydrochlorothiazid ist.

4. Lesinurad-Natrium und Allopurinol zur Verwendung nach Anspruch 1, wobei das Thiaziddiuretikum Hydrochlorothiazid ist.

5. Lesinurad oder ein pharmazeutisch unbedenkliches Salz davon zur Verwendung bei der Behandlung von Gicht in einem Patienten, der ebenfalls mit einem Thiaziddiuretikum und mit Allopurinol behandelt wird, wobei Behandlung ohne Lesinurad oder ein pharmazeutisch unbedenkliches Salz davon nicht ausreichend wäre.

6. Lesinurad-Natrium zur Verwendung nach Anspruch 5.

7. Lesinurad oder ein pharmazeutisch unbedenkliches Salz davon zur Verwendung nach Anspruch 5, wobei das Thiaziddiuretikum Hydrochlorothiazid ist.

8. Lesinurad-Natrium zur Verwendung nach Anspruch 6, wobei das Thiaziddiuretikum Hydrochlorothiazid ist.

## Revendications

1. Lesinurad ou un sel pharmaceutiquement acceptable de celui-ci et allopurinol pour utilisation dans le traitement de la goutte chez un patient également traité avec un diurétique thiazidique.

2. Lesinurad de sodium et allopurinol pour utilisation selon la revendication 1.

3. Lesinurad ou un sel pharmaceutiquement acceptable de celui-ci et allopurinol pour utilisation selon la revendication 1, dans lequel le diurétique thiazidique est l'hydrochlorothiazide.

4. Lesinurad de sodium et allopurinol pour utilisation selon la revendication 1, dans lequel le diurétique thiazidique est l'hydrochlorothiazide.

5. Lesinurad ou un sel pharmaceutiquement acceptable de celui-ci pour utilisation dans le traitement de la goutte chez un patient également traité avec un diurétique thiazidique et avec l'allopurinol lorsqu'un traitement sans Lesinurad ou un sel pharmaceutiquement acceptable de celui-ci n'est pas adéquat.

6. Lesinurad de sodium pour utilisation selon la revendication 5.

7. Lesinurad ou un sel pharmaceutiquement acceptable pour utilisation selon la revendication 5, dans lequel le diurétique thiazidique est l'hydrochlorothiazide.

8. Lesinurad de sodium pour utilisation selon la revendication 6, dans lequel le diurétique thiazidique est l'hydrochlorothiazide.
